# EUROPÄISCHE PATENTSCHRIFT

(11) **EP 3 297 986 B1**
(45) Veröffentlichungstag und Bekanntmachung des Hinweises auf die Patenterteilung: **10.07.2019**
(21) Anmeldenummer: 16724369.0
(22) Anmeldetag: 18.05.2016
(51) Int. Cl.: C07C 303/32, C07C 309/10, C11D 1/00, C25D 3/04, C25D 3/12, C25D 3/32, C25D 3/22, C25D 3/38, C25D 3/56

(54) **BETA-NAPHTHOLETHERSULFONATE, VERFAHREN ZU DEREN HERSTELLUNG UND DEREN VERWENDUNG ALS GLANZVERBESSERER**
BETA NAPHTHOLETHER SULFONATES, METHOD FOR THEIR PREPARATION AND THEIR USE AS GLOSS IMPROVERS
BÉTA-NAPHTALÈNE SULFONATE, SON PROCÉDÉ DE FABRICATION ET D'UTILISATION EN TANT QU'AMPLIFICATEUR DE BRILLANCE

(30) Priorität: 22.05.2015 EP 15168931
(43) Veröffentlichungstag der Anmeldung: 28.03.2018
(73) Patentinhaber: BASF SE, 67056 Ludwigshafen am Rhein (DE)
(72) Erfinder: MAITRO-VOGEL, Sophie, 68199 Mannheim (DE); LORENZ, Michael, 67061 Ludwigshafen (DE); URBAN, Tobias, 64625 Bensheim (DE); KOEHLER, Silke Annika, 68167 Mannheim (DE)
(86) Internationale Anmeldenummer: PCT/EP2016/061065
(87) Internationale Veröffentlichungsnummer: WO 2016/188806

(56) Entgegenhaltungen:
- EP-A1- 0 240 871
- EP-A1- 0 298 296
- JP-A- S6 017 091

## Beschreibung

Die vorliegende Erfindung betrifft beta-Naphtholethersulfonsäuren oder deren Salze, Gemische davon, wässrige Lösungen sowie Elektrolyte diese enthaltend. Die Erfindung betrifft weiterhin Verfahren zu deren Herstellung und die Verwendung dieser.

Sowohl Alkylethersulfonate als auch Arylethersulfonate sind seit langem bekannte Substanzen, die in der galvanischen Metallabscheidung eingesetzt werden. Diese sind z.B. in EP 0 173 832 A2, WO 2012/022689 A1 und EP 0 298 296 A1 ausführlich beschrieben.

So beschreibt EP 0 173 832 A2 in allgemeiner Form Sulfate und Sulfonate von ethoxyliertem beta-Naphthol, wobei ein Ethoxylierungsgrad von 6 bis 60 Ethylenoxyeinheiten sowie im Falle von Sulfonaten eine Vielzahl von Spacerverbindungen zwischen der letzten Ethylenoxyeinheit und der Sulfonatgruppe vorgeschlagen werden. Diese Verbindungen werden in sauren Zinkbädern eingesetzt und sollen in diesen zu einem hohen Trübungspunkt beitragen, um eine einwandfreie Metallabscheidung ohne Schaumentwicklung zu ermöglichen. Als Glanzbildner werden aromatische Ketone und aromatische Aldehyde, wie zum Beispiel Benzalaceton und o-Chlorbenzalaceton, aufgeführt.

WO 2012/022689 A1 befasst sich mit Elektrolyten für die Abscheidung von Bronzelegierungen auf Gebrauchsgütern und industriellen Gegenständen. Diese sollen als Ersatz für nickelhaltige Veredelungsschichten dienen, mit denen solche Gebrauchsgüter in galvanischen Trommel- oder Gestellbeschichtungsverfahren kostengünstig zu allergenfreien, ansehnlichen Erzeugnissen veredelt werden können. Bei der Erzeugung dieser Bronzeschichten für die Elektronikindustrie sind die Lötbarkeit der resultierenden Schicht und gegebenenfalls ihre mechanische Haftfestigkeit die entscheidenden Eigenschaften der zu erzeugenden Schicht. Weiterhin wird in WO 2012/022689 A1 ausgeführt, dass das Aussehen der Schichten für die Anwendung in diesem Bereich in der Regel weniger bedeutsam als ihre Funktionalität ist. Für die Erzeugung von Bronzeschichten auf Gebrauchsgütern ist dagegen die dekorative Wirkung (Glanz und Helligkeit) neben der langen Haltbarkeit der resultierenden Schicht bei möglichst unverändertem Aussehen der wesentliche Zielparameter. Die in WO 2012/022689 A1 vorgeschlagenen Elektrolyte enthalten neben Alkylsulfonsäuren weiterhin ionische Benetzungsmittel in Form von Salzen sulfonierter oder sulfatierter aromatischer Alkylarylether sowie darüber hinaus Komplexmittel und Dialkylthioetherderivate.

In EP 0 298 296 A1 werden ebenfalls beta-Naphtholderivate als Netzmittel, jedoch in Form von Polyalkylenglykol-naphthyl-3-sulfonpropyldiethern und deren Salze für die Galvanotechnik offenbart. Diese Verbindungen sollen insbesondere dazu dienen, den Glanzbildner in Lösung zu halten, die Kathodenoberfläche zu benetzen und die Bildung von sogenannten Wasserstoffporen zu verhindern, die dann entstehen, wenn sich Wasserstoff kathodisch zusammen mit dem abscheidenden Metall, wie Zink, Cadmium, Kupfer, Silber und dergleichen, an der kathodischen Metalloberfläche festsetzt.

Ähnliche Verbindungen zu denjenigen aus EP 0 298 296 A1 werden in EP 1 760 173 A2 zur Abscheidung matter Zinkschichten beschrieben. Die Abscheidung kann sowohl sauer als auch alkalisch erfolgen.

Besonders kurzkettige beta-Naphtholethoxylatsulfonate sind aus US 2,115,192 A bekannt.

Schließlich sind Arylethersulfonate auch kommerziell beispielsweise von der Firma Raschig, Ludwigshafen (DE), unter der Marke RALUFON® NAPE 14-90 erhältlich.

Trotz der bereits im Stand der Technik bekannten Verbindungen für die galvanische Abscheidung besteht ein Bedarf an neuen Verbindungen, die in Bezug auf diese Abscheidung verbesserte Eigenschaften, insbesondere bei der Glanzbildung und hier insbesondere in Bezug auf den Glanzgrad sowie die Glanzstreuung, aufweisen können.

Eine Aufgabe der vorliegenden Erfindung besteht somit darin, solche Verbindungen bereitzustellen.

Die Aufgabe wird gelöst durch die Bereitstellung von beta-Naphtholethersulfonsäuren oder deren Salze mit der allgemeinen Formel (I)

R-O-(AO)ₙ-CH₂-CH₂-S(O)₃M (I),

wobei
R ein Naphth-2-yl Rest darstellt, der unsubstituiert oder mit einem oder mehreren Resten R¹ substituiert ist;
R¹ C₁₋₄ Alkyl ist;
n eine ganze Zahl von 3 bis 25 darstellt;
jedes AO unabhängig voneinander ausgewählt aus einer der Gruppen CH₂-CH₂-O, CH(CH₃)-CH₂-O oder CH₂-CH(CH₃)-O ist und
M für H, Li, Na, K, ½ Mg, ½ Ca, ½ Sr, ½ Ba oder N(R²)₄ steht, wobei jedes R² unabhängig voneinander H, C₁₋₄ Alkyl, Phenyl oder Benzyl bedeutet.

Überraschenderweise wurde gefunden, dass solche Verbindungen bei der galvanischen Abscheidung einen Beitrag zu verbesserten Glanzbildungseigenschaften (insbesondere in Bezug auf Glanzgrad und Glanzstreuung) liefern können.

Dementsprechend handelt es sich um beta-Naphtholethersulfonsäuren oder Salze davon, bei denen an unsubstituiertes oder substituiertes beta-Naphthol n-fach Alkoxygruppen angelagert sind und wobei sich an die letzte Alkoxygruppe eine Ethylengruppe anschließt, an deren anderem Ende sich die SO3M-Gruppe befindet. Sofern M=H ist, handelt es sich um die Sulfonsäuren. Für den Fall, dass M= Li, Na, K, ½ Mg, ½ Ca, ½ Sr, ½ Ba oder N(R²)₄ ist, handelt es sich um eines ihrer Salze.

Für den Fachmann ist selbstverständlich, dass für zweiwertige Metalle, wie Mg, formal nur die Hälfte der Ladung des Metalls für den Ladungsaustauch mit dem Anion erforderlich ist. Dementsprechend entspricht dies einer Formel [R-O-(AO)ₙ-CH₂-CH₂-S(O)₃]₂M für die zweiwertigen Metalle Mg, Ca, Sr und Ba.

Neben den einwertigen Metallen Li, Na, K ist weiterhin ein Ammoniumsalz (M=N(R²)₄) möglich. Der Rest R² bedeutet hierbei jeweils unabhängig H, C₁₋₄ Alkyl, Phenyl oder Benzyl.

Im Rahmen der vorliegenden Erfindung bedeutet "C₁₋₄ Alkyl" eine verzweigte oder unverzweigte, gesättigte Kohlenwasserstoffkette mit einem bis vier Kohlenstoffatome. Hierbei sind zu nennen: Methyl, Ethyl, n-Propyl, i-Propyl, n-Butyl, sec-Butyl, tert.-Butyl, insbesondere Methyl. Die vier Reste R² können gleich oder verschieden sein, vorzugsweise sind diese gleich. Sofern diese verschieden sind, können als Beispiele Methylammonium, Dimethylammonium, Trimethylammonium, Ethylammonium, Diethylammonium oder Methyldiethylammonium genannt werden. Bei gleichen Resten sind zu nennen Ammonium (NH₄₊) oder Tetramethylammonium. Es ist selbstverständlich, dass in Formel (I) die Ladungen nicht gezeigt sind, da diese sich ausgleichen. Dementsprechend tritt anstelle des Ammoniumkations in der Formel M = NH₄.

In Formel (I) bedeutet R einen Naphth-2-yl Rest, der unsubstituiert oder mit einem oder mehreren Resten R¹ substituiert sein kann. Dementsprechend kann R in einer Ausführungsform unsubstituiert sein. In einer anderen Ausführungsform ist R substituiert. Die Anzahl der Substituenten kann 1, 2, 3, 4, 5, 6 oder 7 betragen. Vorzugsweise beträgt die Anzahl der Substituenten 1, 2 oder 3, weiter bevorzugt 1. Diese können gleich sein oder diese sind unterschiedlich. Vorzugsweise sind diese gleich. Ein bevorzugter Substituent ist Methyl. Bevorzugt ist jedoch, wenn R ein unsubstituierter Naphth-2-yl Rest ist.

In Formel (I) gibt n die Anzahl der Alkoxygruppen AO an. Diese liegt im Bereich von 3 bis 25. Dementsprechend können mindestens drei und höchstens 25 AO-Einheiten vorhanden sein. Für eine Einzelverbindung handelt es sich bei n um eine ganze Zahl. Selbstverständlich können auch mehrere Verbindungen der Formel (I) im Gemisch vorliegen. In diesem Fall kann sich eine mittlere Zahl n ergeben, die eine rationale Zahl darstellt, aber dennoch im Bereich von 3 bis 25 liegt.

Die Zahl n liegt im Bereich von 3 bis 25. Vorzugsweise ist n eine Zahl von 6 bis 20. Besonders bevorzugt ist n eine Zahl im Bereich von 8 bis 15 und insbesondere handelt es sich bei n um die Zahl 11.

Bei den Alkoxygruppen AO handelt es sich um Ethylenoxygruppen (CH₂-CH₂-O, EO) oder Propylenoxygruppen (PO) (CH(CH₃)-CH₂-O (2-PO) oder CH₂-CH(CH₃)-O (1-PO)). Für den Fachmann ist klar, dass die Orientierung der Gruppen derart erfolgt, dass das Sauerstoffatom der Oxygruppe jeweils an ein Kohlenstoffatom der nächsten Gruppe anschließt, so dass Etherfunktionalitäten und keine Peroxygruppen gebildet werden. Im Fall von Propylenoxygruppen ist generell bevorzugt, wenn diese von nur einem der beiden Isomeren gebildet werden. Hierbei ist das Isomer CH₂-CH(CH₃)-O bevorzugt.

Die Zahl n kann als Summe aus k+l erhalten werden, wobei k die Anzahl der CH₂-CH₂-O Gruppen und l die Anzahl der beiden Gruppen CH(CH₃)-CH₂-O und CH₂-CH(CH₃)-O ist. Hierbei können dementsprechend k und l Werte von 0 bis 25 annehmen, wobei die Summe k+l im Bereich von 3 bis 25 liegt. Für bevorzugte Bereiche von n gilt das Gleiche für k und l analog.

Dementsprechend können unterschiedliche Gruppen EO und 1-PO; EO und 2-PO; 1-PO und 2-PO; EO, 1-PO und 2-PO oder gleiche Gruppen EO, 1-PO oder 2-PO vorliegen. Bei den unterschiedlichen Gruppen ist EO und 1-PO bevorzugt. Bei den gleichen Gruppen ist EO bevorzugt.

Sofern unterschiedliche Gruppen auftreten, können diese alternierend, blockweise oder zufällig angeordnet sein, sofern die Anzahl n dies zulässt. Bei einer alternierenden Gruppe wechseln sich die unterschiedlichen AO Gruppen ab, beispielsweise EO, 1-PO, EO, 1-PO,..., während eine blockweise Anordnung vorsieht, dass mindestens eine AO Gruppe mindestens zwei benachbarte AO-Einheiten aufweist, beispielsweise 1-PO, 1-PO, EO. Bei einer zufälligen Anordnung ist kein Muster zu erkennen.

Sofern unterschiedliche AO Gruppen vorhanden sind, ist es bevorzugt, wenn sich zunächst an die Naphtholgruppe eine oder zwei 1-PO Gruppen anschließen und dann ausschließlich EO Gruppen folgen. Dementsprechend ist bevorzugt, wenn l = 1 oder 2 und k 1 bis 23 mit der angegebenen Reihenfolge bedeuten und l+k=n, also l+k = 3-25. Für bevorzugte n ist k entsprechend anzupassen, während l = 1 oder 2 ist.

In einer bevorzugten Ausführungsform sind unterschiedliche AO Gruppen vorhanden, wobei k>0 und l>0 ist. Dementsprechend sind EO und PO (bevorzugt 1-PO) Gruppen vorhanden. Vorzugsweise sind mindestens 50 mol-% EO Gruppen vorhanden. Weiterhin ist bevorzugt, dass k>l ist. Dementsprechend sind mehr EO Gruppen als PO Gruppen vorhanden, was einem molaren Anteil von mehr als 50 mol-% bezogen auf alle AO Gruppen entspricht. Weiterhin ist ein Anteil von mindestens 80 mol-% EO bevorzugt.

In einer alternativen bevorzugten Ausführungsform ist nur eine Gruppe AO vorhanden. Hierbei ist bevorzugt, dass AO ausschließlich für EO (CH₂-CH₂-O; k=n, l=0) steht.

Wie bereits eingangs ausgeführt wurde, handelt es sich bei den Verbindungen gemäß allgemeiner Formel (I) um Säuren (M=H) oder deren Salze (M= Li, Na, K, ½ Mg, ½ Ca, ½ Sr, ½ Ba oder N(R²)₄). Bevorzugt sind aufgrund ihrer Wasserlöslichkeit jedoch die Salze. Hierbei sind weiter bevorzugt die einwertigen Kationen und innerhalb dieser Gruppe sind besonders bevorzugt Na und K, insbesondere Na.

Ganz besonders bevorzugt ist das Salz der Formel: 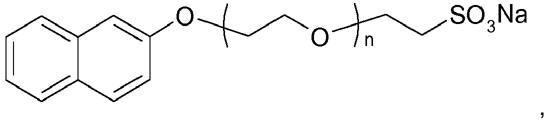 wobei n=11.

Dieses leitet sich aus der Formel (I) her, in dem R für einen unsubstituierten Naphth-2-yl Rest, n für 11, AO für EO, und M für Na steht.

Erfindungsgemäß können die Verbindungen der allgemeinen Formel (I) als Einzelverbindungen oder im Gemisch eingesetzt werden. Sofern ein Gemisch vorliegt, können sich die Verbindungen im Rest R, bei der Zahl n in der Natur der Gruppe AO und/oder in M unterscheiden. Gemische ergeben sich insbesondere durch die Herstellung, bei der entweder bereits Gemische eingesetzt oder während der Herstellung erzeugt werden. Sofern ein Gemisch vorliegt, kann durch bekannte Trennverfahren, wie der Hochdurchsatzchromatographie, eine bestimmte Einzelverbindung erhalten werden. Da dies jedoch kostenaufwändig ist, werden im Rahmen der vorliegenden Erfindung Gemische aus mehreren erfindungsgemäßen Sulfonsäuren oder derer Salzen eingesetzt. Dementsprechend können unterschiedliche Sulfonsäuren, unterschiedliche Salz und/oder unterschiedliche Sulfonsäuren und Salze eingesetzt werden.

Üblicherweise erhält man als Ausgangsmaterial ein Gemisch aus Naphtholalkoxylaten mit unterschiedlichem Alkoxylierungsgrad. Dementsprechend ist bevorzugt, wenn sich die Verbindungen lediglich in der Anzahl n der AO Gruppen unterscheiden.

Ein weiterer Gegenstand der vorliegenden Erfindung ist eine wässrige Lösung enthaltend ein erfindungsgemäßes Salz der Sulfonsäure oder ein erfindungsgemäßes Gemisch von Salzen. Im Rahmen der vorliegenden Erfindung liegt eine wässrige Lösung vor, wenn lediglich Wasser als Lösemittel dient oder zumindest mehr als die volumenbezogene Hälfte aller Lösemittel Wasser ist. Sofern andere Lösemittel vorhanden sind, sind diese vorteilhafterweise bei Raumtemperatur und Normaldruck mit Wasser mischbar. Generell ist jedoch ausschließlich Wasser bevorzugt.

Ein weiterer Gegenstand der vorliegenden Erfindung ist ein Elektrolyt zur galvanischen Metallabscheidung enthaltend
(A) ein erfindungsgemäßes Salz der Sulfonsäure oder ein erfindungsgemäßes Gemisch von Salzen
(B) mindestens ein Metallsalz zur Abscheidung des entsprechenden Metalls und
(C) gegebenenfalls zumindest ein von (A) und (B) unterschiedliche Komponente für die Metallabscheidung.

Im Rahmen der vorliegenden Erfindung bezeichnet der Begriff "Basiselektrolyt" zur galvanischen Metallabscheidung einen Elektrolyten ohne Komponente (A).

Die Komponente (A) wird von einem oben näher erläuterten Salz einer Sulfonsäure entsprechend Formel (I) oder einem diesbezüglichen Gemisch gebildet. Diese wird üblicherweise in Form einer erfindungsgemäßen wässrigen Lösung dem Basiselektrolyten zugesetzt. Dementsprechend handelt es sich bei dem Elektrolyten ebenfalls um eine wässrige Lösung, so dass das bereits Ausgeführte zu der erfindungsgemäßen wässrigen Lösung hier analog gilt.

Bei der Komponente (B) handelt es sich um mindestens ein Metallsalz zur Abscheidung des entsprechenden Metalls. Dementsprechend kann nur ein Salz vorhanden sein, was bevorzugt ist. Weiterhin können mehrere Salze, also beispielsweise zwei, drei oder vier Salze, vorhanden sein, die sich im Metallkation oder im Anion oder sowohl im Kation als auch im Anion unterscheiden.

Geeignete Metallsalze, die bei der Metallabscheidung als Quelle zur Metallabscheidung dienen, sind dem Fachmann geläufig. Da diese in dem Elektrolyt in gelöster Form vorliegen, ist die Wahl des Anions lediglich derart zu wählen, dass die Löslichkeit des Salzes gewährleistet ist und das Anion bei der elektrochemischen Abscheidung diese nicht negativ beeinflusst. Die Wahl des Metallsalzes kann auch vom verwendet pH Wert abhängen. Bei dem Metallsalz handelt es sich vorzugsweise um ein gängiges durch wässrige galvanische Abscheidung abscheidbares Metallsalz, insbesondere Metallsalze der Metalle Zink, Zinn, Kupfer, Nickel, Chrom oder Mischungen solcher Salze, insbesondere ein Zinksalz, wie beispielsweise Zinkchlorid; dementsprechend weist der Elektrolyt beispielhaft unter anderem Zinkionen und Chloridionen auf. Dementsprechend ist das abzuscheidende Metall vorzugsweise Zink, Zinn, Kupfer, Nickel, Chrom oder Legierungen davon, insbesondere Zink.

Bei Komponente (C) handelt es sich um zumindest ein von (A) und (B) unterschiedliches Substanz für die Metallabscheidung, insbesondere übliche Additive, insbesondere organische Additive. Solche Additive sind dem Fachmann bekannt. Komponente (C) ist vorhanden oder nicht vorhanden. Sofern diese vorhanden ist, kann diese ein oder mehrere, wie zwei, drei oder vier Additive aufweisen. Solche Additive für die elektrochemische Metallabscheidung sind dem Fachmann bekannt. Beispielsweise wird die Metallabscheidung in M. Schlesinger, M. Paunovic (Ed.), Modern Electroplating, John Wiley & Sons, Inc., Hoboken, New Jerey (US), 2010 beschrieben. Insbesondere wird in Kapitel 10 die Zinkabscheidung behandelt.

Typische Additive sind beispielsweise nichtionische Tenside wie beispielsweise Alkylethoxylate wie z.B. C10-Oxoalkolhol + 11 EO, Dispergatoren wie beispielsweise Naphthalinsulfonsäure-Kondensationsprodukt, Na-Salz z.B. Tamol und oder Glanzbildner, wie beispielsweise Benzalaceton, Natriumbenzoat. Bei den Glanzbildnern kann unterschieden zwischen solchen die als primäre Glanzbildner verwendet werden und denen, die als Träger zur Benetzung dienen und zur Solubilisierung der primären Glanzbildner beitragen. Beispiele solcher Träger sind Polyalkohole, Polyamine, Fettalkohole, Polyglykolether, und quartäre ammoniumverbindungen. Typische primäre Glanzbildner sind aliphatische, aromatische oder heterozyklische Carbonylverbindungen.

Im Rahmen der vorliegenden Erfindung werden beide Typen von Glanzbildnern zusammen vereinfachend als "Glanzbildner" bezeichnet.

Darüber hinaus sind typische Bestandteile der Komponenten (C) beispielsweise Leitsalze wie Kaliumchlorid und Puffer wie Borsäure, die verwendet werden können.

Der pH-Wert des erfindungsgemäßen Elektrolyten liegt, beispielsweise durch Zugabe von Säuren, im sauren Bereich. Dieser wird vorzugsweise so eingestellt, dass ein Bereich von 4 bis 6,5, bevorzugt ein Bereich von 5 bis 6. Dementsprechend ist eine saure Abscheidung bevorzugt.

Der erfindungsgemäße Elektrolyt wird bevorzugt in einem Bereich von 20°C bis 40°C, bevorzugt um 23°C temperiert. Es ist aber auch in Bereich von 15 °C bis 55 °C möglich. Es kann eine Stromdichte eingestellt werden, die im Bereich 0,01 Ampere pro Quadratdezimeter [A/dm2] bis 15 A/dm2 liegt und die abhängig ist von der Art der Beschichtungsanlage. Ebenso kann die Zellspannung üblicherweise von 1 bis 12 V variieren.

Ein weiterer Gegenstand der vorliegenden Erfindung ist ein Verfahren zur Herstellung einer erfindungsgemäßen Sulfonsäure oder deren Salzes, die Schritte enthaltend:
(a) Umsetzung einer Verbindung der Formel R-O-(AO)ₙ₊₁-H mit Phosgen zu einer Verbindung R-O-(AO)ₙ-CH₂-CH₂-Cl und
(b) Umsetzung der Verbindung R-O-(AO)ₙ-CH₂-CH₂-Cl mit MHSO3 zu einer Verbindung der allgemeinen Formel (I), wobei die Variablen R, AO, n und M die oben angegebene Bedeutung aufweisen.

Ein typisches beispielhaftes Herstellverfahren im kontinuierlich gefahrenen Reaktor ("contineous reactor") über den oben beschriebenen zweistufigen Prozess wird anhand der nachfolgenden Reaktionsgleichung angegeben, wobei Cyanex ein handelsüblicher Katalysator ist:

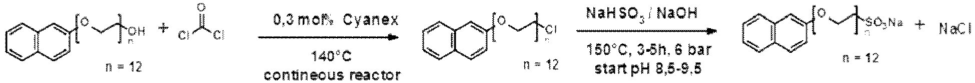

Die Umsetzung von Polyetheralkoholen allgemein in die entsprechenden Chloride durch eine Umsetzung mit Phosgen ist beispielsweise in EP 0 514 683 A1 und EP 0 240 871 A1 beschrieben.

Diese Art der Umsetzung (Schritt (a)) kann sowohl batchweise als auch kontinuierlich betrieben werden. Die Reaktion wird üblicherweise bei Normaldruck durchgeführt, kann aber auch in Druckapparaturen für Phosgenierungen durchgeführt werden. Für die Reaktion können die in EP 0 514 683 A1 und EP 0 240 871 A1 beschriebenen Katalysatoren verwendet werden, nämlich aliphatische, cycloaliphatische oder cyclisch/aliphatische Phospinoxide oder Alkylammoniumchloride. Die Menge an Katalysator kann nach unten bis auf 0,075 mol% bezüglich Alkohol begrenzt werden, nach oben sind nur ökonomische Grenzen gesetzt. Ein Katalysator ist sehr vorteilhaft, da es sonst zur Bildung eines Chlorformiates kommen kann, welches wiederum in das entsprechende Chlorid überführt werden kann. Diese Umsetzung ist allerdings recht zeitaufwendig und führt zu deutlich mehr Nebenprodukten. Die Dosiergeschwindigkeit des Alkohols ist bestimmt durch die Reaktionsgeschwindigkeit der Reaktion. Eine zu schnelle Dosierung führt zu einer unkontrollierbaren Exothermie und zur Bildung von Nebenprodukten. Der Temperaturbereich ist vorzugsweise im Bereich von 20°C bis 180°C. Allerdings ist die Wahl möglichst niedriger Temperaturen vorteilhaft. Die benötigte Temperatur hat direkten Einfluss auf den Überschuss Phosgen, da große Phosgenmengen durch seinen geringen Siedepunkt die Reaktionstemperatur nach oben begrenzen. Man verwendet zweckmäßigerweise einen Überschuss an Phosgen (1,01 - 10 eq. bezüglich Alkohol). Sinnvollerweise soll der Überschuss so gering wie möglich sein. Die Reaktion wird vorteilhaft so gefahren, dass ein leichter Rückfluss an Phosgen zu erkennen ist. Die Reaktion kann in Lösung oder in Reinsubstanz durchgeführt werden, bevorzugt in Reinsubstanz. Als Lösungsmittel können alle gängigen Phosgenierungslösungsmittel verwendet werden, wie Chlorbenzol, Dichlorbenzol, Toluol, Benzol, THF, Dichlormethan etc.

Als Produkt der Umsetzung in Schritt (a) ergibt sich ein Chlorid der Formel R-O-(AO)ₙ-CH₂-CH₂-Cl, wobei R, AO, n die oben angegebene allgemeine oder bevorzugte Bedeutung besitzt. Dieses wird in Schritt (b) weiter umgesetzt.

Es erfolgt in Schritt (b) eine Sulfonierung mit Sulfit. Dementsprechend erfolgt die Umsetzung der Verbindung R-O-(AO)ₙ-CH₂-CH₂-Cl mit MHSO₃ zu einer Verbindung der allgemeinen Formel (I), wobei die Variablen R, AO, n und M die oben angegebene Bedeutung aufweisen.

Auch die Sulfonierung in Schritt (b) mit Hilfe von Sulfit ist generell bekannt. Hierbei sei beispielsweise auf EP 0 026 932 A2 und US 2,989,547 A verwiesen. Vorzugsweise erfolgt die Sulfonierung in Gegenwart von lodidsalzen in einer Finkelsteinreaktion (WO 2008/073956 A2). Vorzugsweise wird das Sulfit, insbesondere Natriumsulfit, in äquimolaren Mengen oder im Überschuss in Bezug auf das Chloridedukt eingesetzt. Bevorzugt ist ein Überschuss, insbesondere ein molarer Überschuss von 0,01 bis 0,1 mol-% in Bezug auf das Edukt.

Die Sulfonierung in Schritt (b) wird vorzugsweise bei einer Temperatur im Bereich von 130 °C bis 180 °C durchgeführt. Vorzugsweise beträgt der pH-Wert 8 bis 10. Dieser kann gegebenenfalls durch die Zugabe des Sulfits gesteuert werden.

Sowohl Alkylethersulfonate als auch Arylethersulfonate sind seit langem bekannte Substanzen, die in der galvanischen Metallabscheidung eingesetzt werden. Diese sind z.B. in EP 0 173 832 A2, WO 2012/022689 A1 und EP 0 298 296 A1 ausführlich beschrieben.

Diese Substanzen erfüllen bei der galvanischen Metallabscheidung generell drei Aufgaben:
1. Benetzung der Oberfläche: Führt zu einer besseren Abscheidung und Haftung der abgeschiedenen Metallschicht.
2. Solubilisierung der sog. Spitzenglanzbildner: Diese sind als Reinstoffe in den wässrigen Medien nicht löslich (z.B. kann dies bei der sauren Zn Abscheidungen Benzalaceton sein).
3. Erhöhung des Trübungspunktes TP: Neben anionischen Tensiden sind auch NIO Tenside und elektrochemisch gebildete Abbauprodukte in den galvanischen Bädern vorhanden, welche den TP senken können.

Des Weiteren erfüllen speziell die Arylethersulfonate einen weiteren Zweck. Aufgrund ihres aromatischen Anteils haben sie zusätzlich zu den oben beschriebenen Eigenschaften noch den Vorteil, dass diese als sog. Grundglanzbildner wirken. D.h. sie beeinflussen auch das Kristallwachstum und steuern so einen Teil zu einer glänzenden Oberfläche bei.

Dementsprechend ist ein weiterer Gegenstand der vorliegenden Erfindung die Verwendung eines erfindungsgemäßen Salzes der Sulfonsäure oder ein erfindungsgemäßes Gemisch von Salzen bei der galvanischen Metallabscheidung, insbesondere der sauren Abscheidung. Bei dem Metall handelt es sich vorzugsweise um Zink, Zinn, Kupfer, Nickel, Chrom oder Legierungen davon, insbesondere um Zink.

Die erfindungsgemäßen Salze der Sulfonsäuren oder deren erfindungsgemäßen Gemische können direkt oder indirekt (bzw. primäre Glanzbildner und Träger) als Glanzverbesserer eingesetzt werden. Direkt bedeutet in dem vorliegenden Zusammenhang, dass diese selbst als Glanzbildner fungieren. Indirekt bedeutet im vorliegenden Zusammenhang, dass diese Additive zur Steigerung der Glanzbildung von Glanzbildnern dienen.

Dementsprechend betrifft ein weiterer Gegenstand die Verwendung eines erfindungsgemäßen Salzes der Sulfonsäure oder ein erfindungsgemäßes Gemisch von Salzen als Glanzverbesserer. Sie können zur Erhöhung des Trübungspunktes, der Glanzstreuung und/oder des Glanzgrades dienen.

### Beispiele

### 1) Umsetzung von beta-Naphtolethoxylat (12-EO-Einheiten) mit Phosgen zum entsprechenden Chlorid

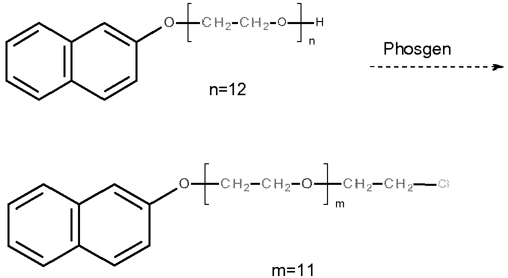

### Versuchsbeschreibung batch:

In einer Rührapparatur mit Stromstörern, Rückflusskühler mit Trockeneiskühlung, Solekühler, Thermometer, Gaseinleitungsrohr, Schutzgasüberlagerung und beheiztem Tropftrichter, werden 0,93 g eines kommerziell erhältlichen Phosphinoxid-Katalysators (Mischung aus 4 trialkylphosphinoxiden, nämlich R₃PO, R₂R'PO, RR'₂PO, R'₃PO (R=n-Octyl, R'=n-Hexyl) vorgelegt und der Rührer eingeschaltet. Der Solekühler wird auf -30°C eingestellt und der Rückflusskühler mit Aceton und Trockeneis bestückt, der Reaktor wird auf 140°C geheizt. Nach Erreichen der Reaktionstemperatur werden 25 g Phosgen zudosiert und ein Rückfluss an Phosgen war zu sehen. Es wurde mit der Dosierung einer Mischung von insgesamt 3417,5 g Beta-Naphtholethoxylat (12 EO) und 4,57 g des oben genannten Phosphinoxidkatalysators begonnen. Es ist eine Abgasentwicklung zu erkennen. Die Edukte wurden so dosiert, dass immer ein Rücklauf an Phosgen und eine Abgasentwicklung zu beobachten war. Die Temperatur bleibt bei konstanten 140 °C. Nach beendeter Zugabe des Beta-Naphtholethoxylates wurde für 1h bei 140°C nachreagiert und anschließend für 22h bei 70°C phosgenfrei gestrippt.

Als Analytik wurden ¹H- und ¹³C-NMR, HPLC, OH Zahl, Phosphorbestimmung angefertigt. Alle Analysen zeigen einen vollständigen Umsatz zum Chlorid.

### Versuchsbeschreibung konti:

Die Syntheseeinheit besteht aus einer Reaktorkaskade von 2 x 1 L Miniplant-Reaktoren aus Glas mit Impellerrührer und Stromstörern, die standgeregelt (auf 400 ml) über Membranpumpen kontinuierlich ineinander überlaufen. Entslztes beta-Naphtholethoxylat (12 EO) und der oben genannte Phosphinoxidkatalysator wurden zuvor gemischt und aus einer beheizten Vorlage mittels intern beheizter Membranpumpe in den Hauptreaktor dosiert. Die Kontrolle erfolgte über eine Waage. Die Reaktoren waren zunächst jeweils mit einem -30/-78 °C Kühlerpaar bestückt. Es schloss eine kontinuierliche Entphosgenierung durch Strippen mit Stickstoff (ca. 150 L/h) in einer beheizten Glockenbodenkolonne (70 °C) an. Das hierbei ausgetriebene Phosgen wurde kondensiert und in den Hauptreaktor zurückgeführt.

Die Anlage wurde mit 130 g/h eines Gemisches aus Beta-Naphtholethoxylat (12 EO) und dem oben genannten Phosphinoxidkatalysator 0,3 mol% und 13 g/h Phosgen über in Summe 30h betrieben. Nach dieser Reaktionszeit war die Anlage im Gleichgewicht, die beiden Reaktoren und die Strippkolonne wurden regelmäßig ca. alle 6h beprobt. Als Analytik wurden ¹H- und ¹³C-NMR und HPLC angefertigt, darüber hinaus bei ausgewählten Proben zusätzlich OH-Zahl und Phosphor.

### 2a) Umsetzung des Chlorides mit Na-Sulfit zum entsprechenden Natrium-beta-Naphtolpolyethoxy(11)sulfonat (Verbindung 1)

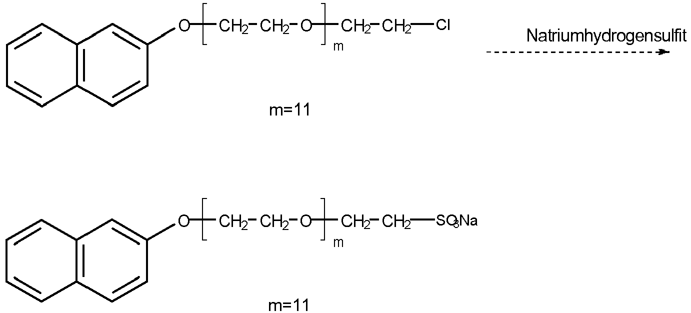

Das oben hergestellte Chlorid (1 mol) wird mit 40%iger Natriumhydrogensulfitlsg (1.1 mol) versetzt und es werden Natrium-beta-Naphtolpolyethoxy(11)sulfonat (2 Gew.-% als wässrige Lösung) und Kaliumiodid (0,05 mol) zugegeben. Die Reaktionslösung wird mit wässriger 10%iger NaOH Lösung auf pH 8.5 eingestellt und anschließend unter Rühren 2h mit Argon entgast. Die zweiphasige Reaktionslösung wird in einen Stahlemailautoklaven oder Hastelloyautoklaven gefüllt, innerhalb von 3h auf 140°C erwärmt und 10h bei dieser Temperatur gehalten. Anschließend wird abgekühlt und man erhält einen homogenen, klaren und bräunlichen Reaktionsaustrag. Der pH des Produktes liegt bei 5,5 - 6,5.

Als Analytik wurden ¹H- und ¹³C-NMR und OH-Zahl angefertigt. Es zeigt sich ein vollständiger Umsatz des Chlorides in 18% Beta-Naphtholethoxylat (12 EO) und 82% Natrium-beta-Naphtholpolyethoxy(11)sulfonat.

### 2b) Umsetzung des Chlorides mit Na-Sulfit zum entsprechenden Natrium-beta-Naphtholpolyethoxy(11)sulfonat (Verbindung 2)

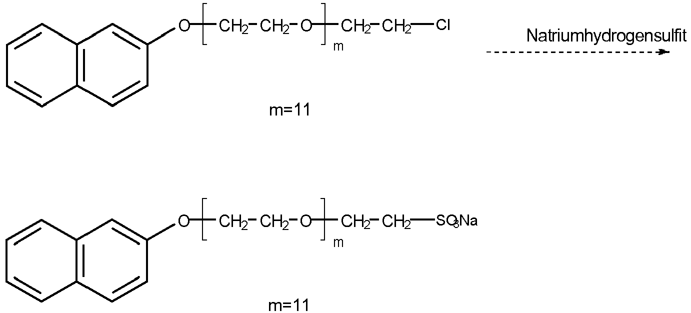

Das oben hergestellte Chlorid (1 mol) wird mit 40%iger Natriumhydrogensulfitlsg (1.1 mol) versetzt und es werden Natrium-beta-Naphtolpolyethoxy(11)sulfonat (2 Gew.-%) zugegeben. Die Reaktionslösung wird mit wässriger 10%iger NaOH Lösung auf pH 8.5 eingestellt und anschließend unter Rühren 2h mit Argon entgast. Die zweiphasige Reaktionslösung wird in einen Stahlemailautoklaven oder Hastelloyautoklaven gefüllt, innerhalb von 3h auf 140°C erwärmt und 15h bei dieser Temperatur gehalten. Anschließend wird abgekühlt und man erhält einen homogenen, klaren und bräunlichen Reaktionsaustrag. Der pH des Produktes liegt bei 6.3.

Als Analytik wurden ¹H- und ¹³C- NMR und OH-Zahl angefertigt. Es zeigt sich ein vollständiger Umsatz des Chlorides in 22% Beta-Naphtholethoxylat (12 EO) und 78% Natrium-beta-Naphtolpolyethoxy(11)sulfonat.

### Vergleichsversuche:

V1: Beta-naphthol-(PO)_{2,5}(EO)₁₄(CH₂)₃SO₃K (Vergleichsverbindung 1) analog Beispiel 6 von EP 0 298 296 A1
V2: Beta-Naphtolethoxylat (12-EO-Einheiten) umgesetzt mit 1,3-Propansulton (Vergleichsverbindung 2) analog V1, jedoch ohne PO und mit der entsprechenden Menge an EO:

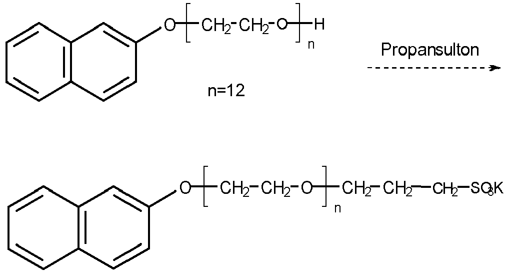

Lugalvan® BNO12+ 1,08äq KOH-Schuppen (88%ig)+ 1äq Propansulton, wässrige Lösung.
Menge: 100g.
Aktivgehalt: ca. 77%, Rest:NIO.
KF-Wasserbestimmung: 6,4%

### Anwendung:

### Testmethode 1 (Glanzstreuung):

Standardmäßig werden Additive für die galvanische Metallabscheidung in der sog. Hull Zelle mit einem definierten Volumen von 250 ml untersucht nach DIN 50957 (Januar 1978). Dabei repräsentiert die linke Seite des Testsubstrates den hohen Stromdichtebereich (SDB), die rechte Seite den niedrigen SDB. Diese Methode ist dem Fachmann bekannt und bedarf keiner näheren Erläuterung.

Als Substrate wurden Stahlbleche mit den Abmessungen 10 x 7 cm verwendet. Diese wurden alkalisch entfettet, in HCl dekapiert und dann in die Hull-Zelle eingebaut. Nach der Beschichtung wurden die Bleche mit Wasser gespült und im Luftstrom getrocknet.

Um den Effekt des neuen Natrium-beta-Naphtolpolyethoxy(11)sulfonates (AES) zu untersuchen, wurde ein Basiselektrolyt verwendet, bei dem lediglich das AES in unterschiedlichen Konzentrationen zugesetzt und verglichen wurde.

Alle nachfolgenden Abscheidungsversuche in der Hull Zelle wurden bei Raumtemperatur, einem Zellstrom von 1A und einer Abscheidedauer von 10 min durchgeführt.

Als Basiselektrolyt wurde folgende Zusammensetzung in Wasser gewählt:
73 g/L ZnCl2
275 g/L KCl
25 g/L H3BO3
2 g/L Natriumbenzoat,
2 g/L Naphtalinsulfonsäure-Kondensationsprodukt, Na-Salz
1 g/L, C10-Oxoalkolhol + 11 EO
1 g/l Thiodiglycol (ethoxyliert)
0,2g/L Benzalaceton

Zu dem Basiselektrolyten werden x g/L Prüfsubstanz (berechnet auf 100%) zugegeben.

### Anwendungsbeispiele:

Im Nachfolgenden wurden zum oben beschriebenen Basiselektrolyt die erfindungsgemäßen Verbindungen in verschiedenen Konzentrationen zugesetzt. Vergleichend dazu wurden zwei Produkte, die ebenfalls Arylethersulfonate (AES) darstellen, jedoch eine -CH₂CH₂CH₂SO₃K-Endgruppe aufweisen, getestet.

### Aus dem oben beschriebenen Elektrolyten wird Zn in der Hull-Zelle abgeschieden und das Abscheidungsbild der Zinkschicht auf dem Testsubstrat bzgl. Glanzgrad bewertet. Bewertet wird das Ausmaß der Bereiche auf dem Blech, welche ausreichenden Glanz, Halbglanz oder eine matte Oberfläche zeigen. Die gesamt Blechlänge beträgt 10 cm.

Es wurden 3 verschiedene Konzentrationen gemessen:
AES 1g/l = Unterdosierung
AES 3g/l = Sollkonzentration
AES 10g/l = Überdosierung
Einsatzmenge im Elektrolyt 3 g/l Aktivsubstanz (= Sollkonzentration)

### Zusammenstellung der Daten:

Glanzbereiche bei Sollkonzentration (3 g/l) wurden vom linken Rand des Bleches gemessen

| | Verbindung 1 | Verbindung 2 | Vergleichsverbindung 1 | Vergleichsverbindung 2 |
|---|---|---|---|---|
| Matter Bereich | 0 - 1,0 cm | 0 - 1,5 cm | 0 - 2,5 cm | 0 - 2 cm |
| Halbglanz | 1,0 - 3,5 cm | 1,5 - 2 cm | 2,5 - 4,5 cm | 2 - 4,5 cm |
| Glanz | 3,5 - 10 cm | 2 - 10 cm | 4,5 - 10 cm | 4,5 - 10 cm |

Überraschenderweise wurde gefunden, dass erfindungsgemäße Verbindungen im Vergleich zu den Vergleichsverbindungen bei gleicher Einsatzkonzentration einen deutlich größeren glänzenden Bereich auf dem Testsubstrat erzeugen.

Dies ist für die Beschichtung von Gütern in der Produktion von Vorteil, da dadurch komplex geformte Teile einheitlicher beschichtet werden können.

Glanzbereiche bei Unterkonzentration (1 g/l) vom linken Rand des Bleches gemessen

| | Verbindung 1 | Vergleichsverbindung 1 | Vergleichsverbindung 2 |
|---|---|---|---|
| Matter Bereich | 0 - 0,8 cm | 0 - 1,0 cm | 0 - 1,5 cm |
| Halbglanz | 0,8 - 3,0 cm | 1,0 - 4,0 cm | 1,5 - 4,5 cm |
| Glanz | 3,0 - 10 cm | 4,0 - 10 cm | 4,5 - 10 cm |

Glanzbereiche bei Überkonzentration (10 g/l) vom linken Rand des Bleches gemessen

| | Verbindung 1 | Vergleichsverbindung 1 | Vergleichsverbindung 2 |
|---|---|---|---|
| Matter Bereich | 0 - 2,0 cm | 0 - 3,5 cm | 0 - 2,0 cm |
| Halbglanz | 2,0 - 3,5 cm | 3,5 - 6,0 cm | 2,0 - 3,5 cm |
| Glanz | 3,5 - 10 cm | 6,0 - 10 cm | 3,5 - 10 cm |

### Testmethode 2 (Glanzgradbestimmung):

Außerdem kann die Glanzgradbestimmung mit dem Gerät: DrLange, REFO 3, Reflektometer (QM-Gerät Nr.G57, ESA/EK, Typ Nr.:LMG136, Geräte Nr.: 1012327) bei einem Messgrad von 85° bestimmt werden. Die Glanzgradbestimmung erfolgt auf einem Stahlblech, verzinkt mit den oben beschriebenen Additiv-Formulierungen enthaltend beta-Naphtolethersulfonate. Die Durchführung der Messung erfolgte nach der Betriebsanweisung des Gerätes REFO 3, jeweils 1cm, 5 cm und 9 cm weg vom Rand gestartet und in hohem Stromdichtebereich (hl -Bereich, siehe DIN 50957, Januar 1987). Der ermittelte Glanzgrad ist der Durchschnittswert von 10 Messungen. Die Standardabweichung beträgt ± 2 Glanzgrade. Je höher der Glanzgrad desto größer ist der Glanz.

| Muster | Verbindung 1 | Vergleichsverbindung 1 | Vergleichs verbindung 2 |
|---|---|---|---|
| Glanzgrad [85°] | Messpunkt vom linken Rand im hl-Bereich: 1 cm / 5cm / 9cm | Messpunkt vom linken Rand im hl-Bereich: 1 cm / 5cm / 9cm | Messpunkt vom linken Rand im hl-Bereich: 1 cm / 5cm / 9cm |
| Zn auf Stahlblech, 1g/l | 95 / 145 / 116 | 75 / 142 / 111 | 87 / 144 / 121 |
| Zn auf Stahlblech, 3g/l | 85 / 142 / 118 | 33 / 110 / 114 | 51 / 138 / 115 |
| Zn auf Stahlblech, 10g/l | 26 / 121 / 120 | 16 / 64 / 109 | 17 / 112 / 116 |
| Zn auf Stahlblech, Grundelektrolyt ohne Additive | 45 / 52 / 57 | 45 / 52 / 57 | 45 / 52 / 57 |

Es ist deutlich, dass der Glanzgrad bei 1 cm und 5 cm bei allen eingesetzten Konzentrationen für die erfindungsgemäße Verbindung besser ist. Bei 9 cm wird die Oberfläche für alle getesteten Produkte matt und der Glanzgrad nimmt ab und ist für alle Produkte ähnlich.

## Patentansprüche

1. Beta-Naphtholethersulfonsäure oder deren Salz mit der allgemeinen Formel (I)
R-O-(AO)n-CH₂-CH₂-S(O)₃M (I),
wobei
R ein Naphth-2-yl Rest darstellt, der unsubstituiert oder mit einem oder mehreren Resten R¹ substituiert ist;
R¹ C₁₋₄ Alkyl ist;
n eine ganze Zahl von 3 bis 25 darstellt;
jedes AO unabhängig voneinander ausgewählt aus einer der Gruppen CH₂-CH₂-O, CH(CH₃)-CH₂-O oder CH₂-CH(CH₃)-O ist und
M für H, Li, Na, K, ½ Mg, ½ Ca, ½ Sr, ½ Ba oder N(R²)₄ steht, wobei jedes R² unabhängig voneinander H, C₁₋₄ Alkyl, Phenyl oder Benzyl bedeutet.

2. Sulfonsäure oder deren Salz nach Anspruch 1, **dadurch gekennzeichnet, dass** R ein unsubstituierter Naphth-2-yl Rest ist.

3. Sulfonsäure oder deren Salz nach Anspruch 1 oder 2, **dadurch gekennzeichnet, dass** n eine ganze Zahl von 6 bis 20 ist.

4. Sulfonsäure oder deren Salz nach einem oder mehreren der Ansprüche 1 bis 3, **dadurch gekennzeichnet, dass** sich n als Summe aus k+l ergibt, wobei k die Anzahl der CH₂-CH₂-O Gruppen und l die Anzahl beiden Gruppen CH(CH₃)-CH₂-O und CH₂-CH(CH₃)-O ist und wobei k>0 und l>0 ist.

5. Sulfonsäure oder deren Salz nach Anspruch 4, **dadurch gekennzeichnet, dass** k>l ist.

6. Sulfonsäure oder deren Salz nach einem oder mehreren der Ansprüche 1 bis 3, **dadurch gekennzeichnet, dass** AO ausschließlich für CH₂-CH₂-O steht.

7. Sulfonsäure oder deren Salz nach einem oder mehreren der Ansprüche 1 bis 6, **dadurch gekennzeichnet, dass** M für Na oder K steht.

8. Salz einer Sulfonsäure nach einem oder mehreren der Ansprüche 1 bis 3 sowie 6 oder 7 der Formel: 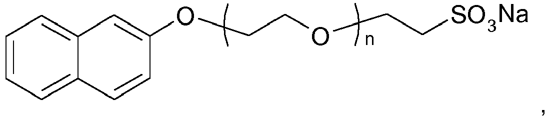 wobei n=11.

9. Gemisch aus mehreren Sulfonsäuren oder derer Salze nach einem oder mehreren der Ansprüche 1 bis 8.

10. Gemisch nach Anspruch 9, wobei die Sulfonsäuren oder deren Salze sich lediglich in der Anzahl n der AO Gruppen unterscheiden.

11. Wässrige Lösung enthaltend ein Salz der Sulfonsäure nach einem der Ansprüche 1 bis 8 oder ein Gemisch von Salzen gemäß Anspruch 9 oder 10.

12. Elektrolyt zur galvanischen Metallabscheidung enthaltend
(A) Salz einer Sulfonsäure nach einem oder mehreren der Ansprüche 1 bis 8 oder ein Gemisch von Salzen nach Anspruch 9 oder 10,
(B) mindestens ein Metallsalz zur Abscheidung des entsprechenden Metalls und
(C) gegebenenfalls zumindest ein von (A) und (B) unterschiedliche Komponente für die Metallabscheidung.

13. Verfahren zur Herstellung einer Sulfonsäure oder deren Salzes nach einem oder mehreren der Ansprüche 1 bis 8, die Schritte enthaltend:
(a) Umsetzung einer Verbindung der Formel R-O-(AO)ₙ₊₁-H mit Phosgen zu einer Verbindung R-O-(AO)ₙ-CH₂-CH₂-Cl und
(b) Umsetzung der Verbindung R-O-(AO)ₙ-CH₂-CH₂-Cl mit MHSO₃ zu einer Verbindung der allgemeinen Formel (I), wobei die Variablen R, AO, n und M die in Anspruch 1 angegebene Bedeutung aufweisen.

14. Verwendung eines Salzes der Sulfonsäure nach einem der Ansprüche 1 bis 8 oder ein Gemisch von Salzen gemäß Anspruch 9 oder 10 bei der galvanischen Metallabscheidung.

15. Verwendung eines Salzes der Sulfonsäure nach einem der Ansprüche 1 bis 8 oder ein Gemisch von Salzen gemäß Anspruch 9 oder 10 als Glanzverbesserer.

## Claims

1. A beta-naphthol ether sulfonic acid or salt thereof having the general formula (I)
R-O-(AO)n-CH₂-CH₂-S(O)₃M (I),
where
R is a naphth-2-yl radical which is unsubstituted or substituted by one or more radicals R¹;
R¹ is C₁₋₄ alkyl;
n is an integer from 3 to 25;
each AO independently of any other is selected from one of the groups CH₂-CH₂-O, CH(CH₃)-CH₂-O or CH₂-CH(CH₃)-O, and
M is H, Li, Na, K, ½ Mg, ½ Ca, ½ Sr, ½ Ba or N(R²)₄, where each R² independently of any other is H, C₁₋₄ alkyl, phenyl or benzyl.

2. The sulfonic acid or salt thereof according to claim 1, wherein R is an unsubstituted naphth-2-yl radical.

3. The sulfonic acid or salt thereof according to claim 1 or 2, wherein n is an integer from 6 to 20.

4. The sulfonic acid or salt thereof according to one or more of claims 1 to 3, wherein n is the sum of k+l, where k is the number of CH₂-CH₂-O groups and 1 is the number of both groups CH(CH₃)-CH₂-O and CH₂-CH(CH₃)-O, and where k > 0 and l > 0.

5. The sulfonic acid or salt thereof according to claim 4, wherein k > 1.

6. The sulfonic acid or salt thereof according to one or more of claims 1 to 3, wherein AO is exclusively H₂-CH₂-O.

7. The sulfonic acid or salt thereof according to one or more of claims 1 to 6, wherein M is Na or K.

8. A salt of a sulfonic acid according to one or more of claims 1 to 3 and 6 and 7, of the formula: 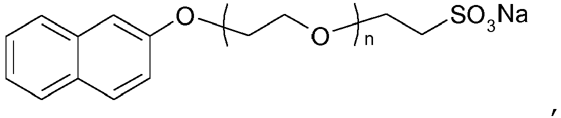 where n = 11.

9. A mixture of a plurality of sulfonic acids or salts thereof according to one or more of claims 1 to 8.

10. The mixture according to claim 9, the sulfonic acids or salts thereof differing only in the number n of AO groups.

11. An aqueous solution comprising a salt of the sulfonic acid according to any of claims 1 to 8 or a mixture of salts according to claim 9 or 10.

12. An electrolyte for electrochemical deposition of metal, comprising
(A) salt of a sulfonic acid according to one or more of claims 1 to 8 or a mixture of salts according to claim 9 or 10,
(B) at least one metal salt for the deposition of the corresponding metal, and
(C) optionally at least one metal deposition component other than (A) and (B).

13. A process for preparing a sulfonic acid or salt thereof according to one or more of claims 1 to 8, comprising the steps of:
(a) reacting a compound of the formula R-O-(AO)ₙ₊₁-H with phosgene to give a compound R-O-(AO)ₙ-CH₂-CH₂-Cl and
(b) reacting the compound R-O-(AO)ₙ-CH₂-CH₂-Cl with MHSO₃ to give a compound of the general formula (I), the variables R, AO, n, and M having the definition stated in claim 1.

14. The use of a salt of sulfonic acid according to any of claims 1 to 8 or a mixture of salts according to claim 9 or 10 in electrochemical deposition of metal.

15. The use of a salt of sulfonic acid according to any of claims 1 to 8 or a mixture of salts according to claim 9 or 10 as a brightness improver.

## Revendications

1. Acide bêta-naphtol-éther-sulfonique ou son sel de formule générale (I)
R-O-(AO)ₙ-CH₂-CH₂-S(O)₃M (I)
dans laquelle
R représente un radical napht-2-yle, qui est non substitué ou substitué avec un ou plusieurs radicaux R¹ ;
R¹ représente alkyle en C₁₋₄ ;
n représente un nombre entier de 3 à 25 ;
les AO sont chacun choisis indépendamment les uns des autres dans le groupe constitué par CH₂-CH₂-O, CH(CH₃)-CH₂-O ou CH₂-CH (CH₃)-O, et
M représente H, Li, Na, K, ½ Mg, ½ Ca, ½ Sr, ½ Ba ou N(R²)₄, les R² signifiant chacun indépendamment les uns des autres H, alkyle en C₁₋₄, phényle ou benzyle.

2. Acide sulfonique ou son sel selon la revendication 1, **caractérisé en ce que** R est un radical napth-2-yle non substitué.

3. Acide sulfonique ou son sel selon la revendication 1, **caractérisé en ce que** n est un nombre entier de 6 à 20.

4. Acide sulfonique ou son sel selon une ou plusieurs des revendications 1 à 3, **caractérisé en ce que** n est la somme de k+l, k étant le nombre de groupes CH₂-CH₂-O et l étant le nombre des deux groupes CH(CH₃)-CH₂-O et CH₂-CH (CH₃)-O, avec k > 0 et l > 0.

5. Acide sulfonique ou son sel selon la revendication 4, **caractérisé en ce que** k > l.

6. Acide sulfonique ou son sel selon une ou plusieurs des revendications 1 à 3, **caractérisé en ce qu'**AO représente exclusivement CH₂-CH₂-O.

7. Acide sulfonique ou son sel selon une ou plusieurs des revendications 1 à 6, **caractérisé en ce que** M représente Na ou K.

8. Sel d'un acide sulfonique selon une ou plusieurs des revendications 1 à 3, ainsi que 6 ou 7, de formule : 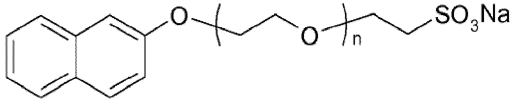 avec n = 11.

9. Mélange de plusieurs acides sulfoniques ou leurs sels selon une ou plusieurs des revendications 1 à 8.

10. Mélange selon la revendication 9, dans lequel les acides sulfoniques ou leurs sels diffèrent uniquement au niveau du nombre n de groupes AO.

11. Solution aqueuse contenant un sel de l'acide sulfonique selon l'une quelconque des revendications 1 à 8 ou un mélange de sels selon la revendication 9 ou 10.

12. Électrolyte pour le dépôt galvanique de métaux, contenant :
(A) un sel d'un acide sulfonique selon une ou plusieurs des revendications 1 à 8 ou un mélange de sels selon la revendication 9 ou 10,
(B) au moins un sel métallique pour le dépôt du métal correspondant, et
(C) éventuellement au moins un composant différent de (A) et (B) pour le dépôt du métal.

13. Procédé de fabrication d'un acide sulfonique ou son sel selon une ou plusieurs des revendications 1 à 8, comprenant les étapes suivantes :
(a) la mise en réaction d'un composé de formule R-O-(AO)ₙ₊₁-H avec du phosgène pour former un composé R-O-(AO)ₙ-CH₂-CH₂-Cl, et
(b) la mise en réaction du composé R-O-(AO)ₙ-CH₂-CH₂-Cl avec MHSO₃ pour former un composé de formule générale (I), les variables R, AO, n et M ayant la signification indiquée dans la revendication 1.

14. Utilisation d'un sel de l'acide sulfonique selon l'une quelconque des revendications 1 à 8 ou d'un mélange de sels selon la revendication 9 ou 10 lors du dépôt galvanique de métaux.

15. Utilisation d'un sel de l'acide sulfonique selon l'une quelconque des revendications 1 à 8 ou d'un mélange de sels selon la revendication 9 ou 10 en tant qu'agent d'amélioration de la brillance.
